# EUROPÄISCHE PATENTANMELDUNG

(11) **EP 4 272 882 A1**
(43) Veröffentlichungstag der Anmeldung: **08.11.2023**
(21) Anmeldenummer: 23168472.1
(22) Anmeldetag: 18.04.2023
(51) Int. Cl.: B09B 3/45, G01N 33/44, G01N 33/00, B29B 17/00

(54) **VORRICHTUNG UND VERFAHREN ZUM ERMITTELN VON BETRIEBSPARAMETERN FÜR DAS ENTGASEN UND/ODER DESODORIEREN VON KUNSTSTOFFPARTIKELN**

(30) Priorität: 04.05.2022 DE 102022204421
(71) Anmelder: Coperion GmbH, 70469 Stuttgart (DE)
(72) Erfinder: Lang, Klaus-Peter, 88339 Bad Waldsee (DE); Dikreuter, Christian, 88090 Immenstaad (DE)
(74) Vertreter: Rau, Schneck & Hübner Patentanwälte Rechtsanwälte PartGmbB

(57) **Zusammenfassung**

Eine Vorrichtung (1) zum Ermitteln von Betriebsparametern für das Entgasen und/oder Desodorieren von Kunststoffpartikeln umfasst mindestens eine Testkammer (3) zum Entgasen und/oder Desodorieren einer Testmenge von Kunststoffpartikeln wobei jede Testkammer (3) eine Einfüllöffnung zum Einfüllen der Testmenge, eine Entnahmeöffnung (10) zum Entnehmen von Schüttgut, eine Entlüftungsöffnung (6) zum Entlüften der Testkammer (3) aufweist, wobei an eine Zuführöffnung (35) der Testkammer (3) angeschlossen sind eine Druckgasleitung (31) zum Zuführen von Druckgas und ein Heizelement (22) und/oder eine Dampfleitung (32) zum Zuführen von Dampf und ein Dampfgenerator (16).

## Beschreibung

Der Inhalt der deutschen Patentanmeldung DE 10 2022 204 421.5 wird durch Bezugnahme hierin aufgenommen.

Die Erfindung betrifft eine Vorrichtung und ein Verfahren zum Ermitteln von Betriebsparametern für das Entgasen und/oder Desodorieren von Kunststoffpartikeln.

Es ist bekannt, dass Kunststoffpartikel, flüchtige Bestandteile, insbesondere Monomere, Feuchtigkeit, Gase, insbesondere Luft, Lösungsmittel, Gerüche und Aromen wie beispielsweise Limonene und/oder flüchtige organische Verbindungen (VOC) aufweisen können. Als Kunststoffpartikel werden je nach Partikelgröße und/oder Partikelform beispielsweise Kunststoffgranulat, Kunststoffflocken und Kunststoffpulver unterschieden. Der Einfachheit halber werden die Kunststoffpartikel im Folgenden als Schüttgut bezeichnet. Um die flüchtigen Bestandteile zu entfernen, kann das Schüttgut beispielsweise in einer Mischeinrichtung gemäß DE 10 2012 206 017 A1 entgast und/oder desodoriert werden. Beim Entgasen und/oder Desodorieren des Schüttguts wird dieses mit einem Gas, insbesondere Luft, umspült, um die flüchtigen Bestandteile zu entfernen. Die dafür erforderlichen Betriebsparameter wie Temperatur, Druck, Luftmenge, Feuchte und Entgasungsdauer werden typischerweise erfahrungsgemäß und/oder experimentell ermittelt und sind nicht zuletzt von dem zu entgasenden Schüttgut abhängig. Die Ermittlung geeigneter Betriebsparameter ist aufwendig. Wechselnde Güten des zu verarbeitenden Schüttguts erhöhen den Aufwand für die Bestimmung der Betriebsparameter für Entgasung und/oder Desodorierung enorm. Insbesondere Schüttgut aus Kunststoffrezyklat kann besonders häufig wechselnde Güten aufweisen. Das Entgasen und/oder Desodorieren von Kunststoff-Schüttgut ist zeitaufwendig, energieintensiv und damit kostenintensiv.

Es ist eine Aufgabe der vorliegenden Erfindung, das Entgasen und/oder Desodorieren von Schüttgut zu vereinfachen und insbesondere den Aufwand für die Ermittlung entsprechender Betriebsparameter zu reduzieren.

Die Aufgabe ist erfindungsgemäß gelöst durch eine Vorrichtung mit den im Anspruch 1 angegebenen Merkmalen und durch ein Verfahren mit den im Anspruch 15 angegebenen Merkmalen.

Erfindungsgemäß wurde erkannt, dass relevante Betriebsparameter für das Entgasen und/oder Desodorieren von Kunststoffpartikeln mittels einer geeigneten Testmenge in einer dafür vorgesehenen Vorrichtung ermittelt werden können. Die Vorrichtung ist insbesondere ein Teststand, der insbesondere kompakt und insbesondere transportabel, also mobil ausgeführt ist. Mobil bedeutet insbesondere, dass der Teststand selbst beweglich, insbesondere fahrbar ausgeführt sein kann. Dazu kann der Teststand mindestens eine und insbesondere mehrere Fahrrollen und/oder Räder aufweisen. Die Rollen und/oder Räder sind insbesondere an einer Unterseite eines Gestells des Teststands angeordnet und insbesondere höhenverstellbar ausgeführt. Die Rollen und/oder Räder können um eine Vertikalachse drehbar und/oder arretierbar am Teststand angelenkt sein. Um die Handhabung des mobilen Teststands zu verbessern, kann der Teststand einen oder mehrere Handgriffe aufweisen. Der Teststand kann bequem geschoben und/oder gezogen werden. Der mindestens eine Handgriff kann demontierbar ausgeführt sein. Die Ermittlung der Betriebsparameter ist unaufwendig und insbesondere flexibel und /oder bedarfsgerecht möglich.

Mit der Vorrichtung kann, insbesondere vorab, die Testmenge der Kunststoffpartikel aufbereitet und analysiert werden, um die Betriebsparameter, also Entgasungsparameter für ein Aufbereitungsverfahren, zu ermitteln. Diese Betriebsparameter können an einem Entgasungssilo einer Compoundier- und/oder Recyclinganlage eingestellt werden. Zusätzlich oder alternativ kann die Vorrichtung genutzt werden, um neue Anlagen bereits während ihrer Planungsphase für bestimmte Kunststoffpartikel, insbesondere optimal, auszulegen. Eine geeignete Testmenge beträgt zwischen 11 und 100 1, insbesondere zwischen 5 1 und 50 1, insbesondere zwischen 5 1 und 20 1, insbesondere zwischen 8 1 und 15 1 und insbesondere 10 1, also 0,01 m³. Es wurde gefunden, dass dieses Volumen der Testmenge ausreichend ist, um den Entgasungs- und/oder Desodorierungs-Vorgang realitätsgetreu abzubilden, also zu simulieren. Die Testmenge ist also ausreichend groß, sodass Skalierungseffekte gegenüber einer realen Compoundier- und/oder Recyclinganlage vernachlässigbar sind.

Das Schüttgut umfasst Kunststoff-Einzelpartikel, insbesondere Granulat mit einer mittleren Teilchengröße zwischen 0,5 mm bis 6,0 mm, insbesondere 0,7 mm bis 4,0 mm.Kunststoffpartikel können auch Pulverteilchen mit einer mittleren Korngröße zwischen 1 µm und 2.000 µm, insbesondere zwischen 10 µm und 2.000 µm, insbesondere zwischen 50 µm und 2.000 µm, insbesondere zwischen 50 µm und 200 µm, insbesondere zwischen 70 µm und 200 µm und insbesondere zwischen 70 µm und 150 µm, und/oder flockenartige Teilchen, sogenannte Flakes, mit einer mittleren Kantenlänge zwischen 1,0 mm und 30,0 mm, insbesondere zwischen 2,0 mm und 25,0 mm und insbesondere zwischen 5,0 mm und 20,0 mm sein.

Als Kunststoffmaterial dienen insbesondere Polyolefine, insbesondere Polyethylenterephthalat (PET) und/oder Polystyrol (PS).

Es wurde gefunden, dass in einer Testkammer der Vorrichtung Betriebsbedingungen für das Entgasen und/oder Desodorieren eingestellt werden können, die den realen Betriebsbedingungen in einem Silo einer Compoundier- und/oder Recyclinganlage entsprechen. In der Testkammer können also die Betriebsparameter entsprechend einem großen Entgasungssilo simuliert werden, obwohl das Entgasungssilo ein Volumen aufweist, das deutlich größer als das Volumen der Testkammer ist. Insbesondere beträgt das Volumen der Testkammer höchstens das 0,1-fache, insbesondere höchstens das 0,01-fache, insbesondere höchstens das 0,005-fache, insbesondere höchstens das 0,001-fache und insbesondere höchstens das 0,0001-fache des Volumens eines Entgasungssilos.

Die Vorrichtung weist mindestens eine Testkammer auf. Wenn mehrere Testkammern vorgesehen sind, ist es insbesondere möglich, parallel verschiedene Betriebsbedingungen zu simulieren. Dadurch kann der Zeitaufwand für die Bestimmung geeigneter Betriebsbedingungen reduziert werden. Die Testkammern sind insbesondere vergleichbar und insbesondere identisch zueinander ausgeführt. Insbesondere weisen die Testkammern jeweils eine identische Größe auf und ermöglichen insbesondere die Aufnahme von Testmengen identischen Volumens. Die Testkammern können auch Volumina unterschiedlicher Größe aufweisen. Funktional sind die Testkammern insbesondere identisch ausgeführt.

Die realen Betriebsparameter können anhand einer vergleichsweise kleinen Testmenge unaufwendig und schnell ermittelt werden. Die Testkammer weist eine, insbesondere an ihrer oberen Stirnseite angeordnete, Einfüllöffnung auf, um die Testmenge einzufüllen.

Die in der Testkammer angeordnete Testmenge kann mit einem Gas zum Entgasen beaufschlagt werden. Als Gas dient insbesondere Luft, insbesondere Druckluft. Es können aber auch andere Gase verwendet werden, insbesondere Stickstoff. Vorteilhaft ist es, wenn das Druckgas inert ist.

Die Testkammer weist eine Zuführöffnung auf, um Druckgas, insbesondere Druckluft, von einer Druckgasleitung in die Testkammer zuzuführen. Die Vorrichtung ermöglicht die Zuführung von erhitztem Gas in die Testkammer. Ein Heizelement dient insbesondere zum Erhitzen des Gases und ist an die Druckgasleitung angeschlossen. Das Heizelement ist insbesondere als elektrische Heizung ausgeführt, insbesondere als Lufterhitzer. Der Lufterhitzer kann auch für die Beheizung von inerten Gasen, insbesondere Stickstoff, verwendet werden. Bezüglich der Druckgasleitung bildet die Zuführöffnung einen Druckgasanschluss an die Testkammer.

Die Vorrichtung ermöglicht es, die Testmenge in der Testkammer mit heißem Druckgas, insbesondere mit heißer Luft, mit einer Temperatur von Umgebungstemperatur bis zu 150 °C zu beaufschlagen. Insbesondere ermöglicht die Vorrichtung Temperaturbeaufschlagungen von mindestens 50 °C, insbesondere von mindestens 70 °C, insbesondere von mindestens 90 °C, insbesondere von mindestens 100 °C, insbesondere von mindestens 120 °C.

Um verschiedene Feuchtigkeitsgehälter in der Testkammer zu realisieren, kann der Testkammer Dampf, insbesondere Wasserdampf, mittels einer Dampfleitung zugeführt werden. An die Dampfleitung ist ein Dampfgenerator angeschlossen, der Wasser verdampfen kann. Die Dampfleitung ist an die Zuführöffnung der Testkammer angeschlossen. Bezogen auf die Dampfleitung bildet die Zuführöffnung einen Dampfanschluss.

Insbesondere münden die Dampfleitung und die Druckgasleitung in dieselbe Zuführöffnung. Insbesondere münden die beiden Leitungen stromaufwärts der Zuführöffnung ineinander, so dass an die Zuführöffnung nur eine einzige Leitung angeschlossen ist. Dieser an die Zuführöffnung angeschlossene Leitungsabschnitt, der insbesondere ein Teil der Dampfleitung oder der Druckgasleitung ist, wird auch als Begasungsleitung bezeichnet.

Eine Erkenntnis der Erfindung beruht darauf, dass für die Ermittlung der Betriebsparameter in die Testkammer durch die Zuführöffnung erhitztes Druckgas und/oder Wasserdampf zugeführt werden kann.

Die Testkammer weist, insbesondere seitlich, eine Entnahmeöffnung auf, um eine Probemenge der Kunststoffpartikel aus der Testkammer zu entnehmen. Insbesondere bildet die Probenmenge eine Teilmenge der Testmenge. Die Probenmenge weist beispielsweise ein Volumen von 20 ml bis 200 ml auf. Die Entnahme der Probemenge dient zur Ermittlung des Entgasungs- und/oder Desodorierungsfortschritts und damit der Verifizierung der zuvor angewendeten Betriebsparameter. Die Betriebsparameter umfassen insbesondere Temperatur, Druck, Luftmenge, Feuchtigkeitsgehalt und Prozessdauer, also Begasungsdauer. Insbesondere werden mit der Vorrichtung mehrere Versuchsreihen durchgeführt, um die Betriebsparameter zu ermitteln. Die Durchführung eines Versuchs ist unkompliziert durchführbar.

Es wurde gefunden, dass die erfindungsgemäße Vorrichtung kleinbauend, also kompakt ausgeführt sein kann. Insbesondere weist die Testkammer ein Maximalvolumen von höchstens 100 1, insbesondere höchstens 50 1, insbesondere höchstens 20 1 und insbesondere 10 1 auf. Insbesondere ist die Vorrichtung mobil ausgeführt, also transportabel. Die Vorrichtung kann beispielsweise mit einem Kleintransporter transportiert werden. Insbesondere ist die Vorrichtung derart konstruiert, dass überstehende Anbauteile wie Leitungen, Rohre, Kabel und/oder Gehäuseteile für den Transport der Vorrichtung unkompliziert demontierbar sind. Es ist insbesondere möglich, die Vorrichtung unmittelbar zu der Compoundier- und/oder Recyclinganlage zu transportieren und die Betriebsparameter für das jeweils zu fördernde Schüttgut unmittelbar zu ermitteln. Insbesondere kann die Vorrichtung derart ausgeführt sein, dass sie mit gängigen Hebe- und/oder Fördermitteln wie beispielsweise einem Gabelstapler oder Hubwagen angehoben und verfahren werden kann. Dazu kann die Vorrichtung mittels einer, insbesondere standardisierten, Transport-Palette, einer sog. Europalette EPAL, transportiert werden.

Im Transportzustand ist eine Grundfläche der Vorrichtung insbesondere rechteckig oder in einem Rechteck anordenbar, das eine maximale Länge von 1,8 m, insbesondere von höchstens 1,6 m und insbesondere von höchstens von 1,5 m aufweist. Das die Grundfläche der Vorrichtung umgebende Rechteck weist eine maximale Breite von höchstens 1,5 m, insbesondere von höchstens 1,3 m und insbesondere von 1,2 m auf. Eine maximale Höhe der Vorrichtung im Transportzustand beträgt höchstens 1,6 m, insbesondere höchstens 1,5 m und insbesondere höchstens 1,4 m.

Insbesondere ist die Masse der Vorrichtung kleiner als die maximale Tragfähigkeit einer Europalette. Insbesondere beträgt die maximale Masse der Vorrichtung höchstens 2,0 t, insbesondere höchstens 1,5 t und insbesondere höchstens 1,0 t.

Insbesondere weist die Testkammer eine Hauptentlüftung auf. An der Testkammer ist eine Entlüftungsöffnung, insbesondere an ihrer oberen Stirnseite, angeordnet, an die insbesondere eine Entlüftungsleitung anschließbar ist. Die Entlüftungsleitung ist insbesondere als Kaminrohr ausgeführt. Die Entlüftungsleitung ist insbesondere demontierbar an der Entlüftungsöffnung angebracht. Die demontierbare Entlüftungsleitung ermöglicht eine besonders kompakte Bauform der Testkammer. Über die Hauptentlüftung können heiße und insbesondere gesundheitsschädliche Dämpfe, insbesondere PVC-Anteile enthaltende Dämpfe, aus der Testkammer zuverlässig und insbesondere oberhalb einer Arbeitsebene eines Bedieners abgeführt werden. Mit der Entlüftungsleitung kann die Testkammer an eine zentrale Entlüftungseinrichtung angeschlossen werden.

Für die Stromversorgung der Vorrichtung ist insbesondere eine zentrale Spannungsversorgung vorgesehen. Es kann sich dabei um einen entsprechenden elektrischen Anschluss handeln, der eine Verbindung mit einer zentralen Spannungsversorgung, insbesondere einem Leitungsnetz ermöglicht. Alternativ kann ein entsprechendes Gerät, insbesondere ein Generator und/oder ein Akkumulator, an der Vorrichtung vorgesehen sein, das die erforderliche Spannungsversorgung bereitstellt, insbesondere für einen mobilen und insbesondere einen autarken Einsatz der Anlage.

Eine Vorrichtung gemäß Anspruch 2 ermöglicht eine flexible, insbesondere bedarfsgerechte, Anpassung, insbesondere Regulierung der zugeführten Gasmenge und/oder des Gasdruckes in der Testkammer. Ein Gasmengenregulierer ermöglicht insbesondere das Regulieren eines Gasstroms, insbesondere eines Luftstroms, was insbesondere zum Erwärmen oder Warmhalten des Gehäuses der Testkammer dient. Die Gasmengen- und/oder Gasdruckregulierung kann manuell und/oder automatisiert, insbesondere vollautomatisch, erfolgen. Entlang der Gasströmungsrichtung ist der Gasmengenregulierer insbesondere stromabwärts bezogen auf das Heizelement angeordnet. Die Anordnung des Gasmengenregulierers und Heizelement entlang der Gasströmungsrichtung kann auch umgekehrt sein.

Eine Vorrichtung gemäß Anspruch 3 gewährleistet eine zuverlässige Druckgasversorgung. Besonders vorteilhaft ist es, wenn die Druckgasleitung an eine Druckgasquelle in Form eines zentralen Druckluftnetzes anschließbar ist. Ein derartiges Druckluftnetz stellt Druckgas, insbesondere Druckluft, in einem Bereich von 4 bar bis 8 bar zur Verfügung und ist beispielsweise in gewerblichen Anlagen oftmals ohnehin vorhanden. Eine separate Druckgasquelle ist entbehrlich. Vorhandene Ressourcen können unmittelbar genutzt werden.

Alternativ oder zusätzlich ist es möglich, als Druckgasquelle einen Kompressor zu nutzen, der an die Druckgasleitung angeschlossen ist. Ein Kompressor als Druckgasquelle erhöht die Einsatzflexibilität der Vorrichtung und ermöglicht einen autarken Betrieb der Vorrichtung.

Eine Entlüftungsöffnung gemäß Anspruch 5 ermöglicht einen gefahrlosen Betrieb des Dampfgenerators.

Vorteilhaft ist, wenn der Dampfgenerator eine Entlüftungsöffnung aufweist, an die eine Entlüftungsleitung, insbesondere in Form eines Entlüftungsrohres, angeschlossen ist. Das Entlüftungsrohr ist insbesondere vertikal orientiert und weist eine Länge insbesondere derart auf, um Dampf aus dem Dampfgenerator in einem Höhenabschnitt abzugeben, der oberhalb einer Kopfhöhe eines Bedieners angeordnet ist, insbesondere in einer Höhe von mindestens 2,5 m oberhalb des Bodens. Die Entlüftungsleitung ist für den Transport demontierbar.

Eine an den Dampfgenerator angeschlossene Wasserenthärtungseinheit gemäß Anspruch 5 ermöglicht die Nutzung von kalkhaltigem Trinkwasser, insbesondere eines vorhandenen Trinkwasserleitungsnetzes, für die Dampferzeugung.

Ein Dampfmengenregulierer gemäß Anspruch 6 ermöglicht die veränderlich festlegbare Feuchtezugabe in die Testkammer. Der Dampfmengenregulierer ist manuell oder automatisiert, insbesondere vollautomatisch, betätigbar. Mit dem Dampfmengenregulierer kann gezielt ein Dampfstrom reguliert werden.

Eine anschließbare Wasserquelle gemäß Anspruch 7 stellt die unmittelbare Feuchtezugabe in die Testkammer sicher. Besonders vorteilhaft ist es, wenn ein ohnehin vorhandenes Leitungsnetz zur Wasserversorgung nutzbar ist. Zusätzlich oder alternativ kann die Vorrichtung einen Wassertank und insbesondere eine Pumpe aufweisen, um insbesondere einen autarken Betrieb der Vorrichtung zu ermöglichen.

Eine Entlüftungsleitung gemäß Anspruch 8 gewährleistet eine zuverlässige und gefahrlose Ableitung von Gasen aus der Testkammer. Mittels der mindestens einen Entlüftungsleitung ist die Testkammer zuverlässig an ein insbesondere vorhandenes Entlüftungssystem anschließbar.

Eine Entnahmeeinheit gemäß Anspruch 9 ermöglicht die zuverlässige und insbesondere wiederholgenaue Entnahme einer Schüttgutmenge, um die zuvor in der Testkammer angewendeten Betriebsparameter zu verifizieren. Eine mittels der Entnahmeeinheit entnommene Schüttgutmenge kann näher untersucht und analysiert werden. Besonders vorteilhaft ist es, wenn die Entnahmeeinheit eine absperrbare Entnahmeleitung, insbesondere in Form eines Fallrohrs aufweist. Die Ausführung der Entnahmeeinheit ist zuverlässig, robust und unkompliziert. Insbesondere ist das Fallrohr gegenüber der vertikalen um einen Neigungswinkel geneigt, der insbesondere höchstens 30 °, insbesondere höchstens 20 °, insbesondere höchstens 15 °, insbesondere höchstens 10 ° und insbesondere mindestens 5 ° beträgt.

Eine Steuerungseinheit gemäß Anspruch 10 vereinfacht die Durchführung von Versuchsreihen mit unterschiedlichen Betriebsparametern. Insbesondere gewährleistet die Steuerungseinheit die Durchführung eines Entgasungszyklus mit veränderlich festlegbaren Betriebsparametern, also Temperatur, Druck und/oder Menge des zugeführten Druckgases, Wassergehalt, also Feuchtegehalt, und Entgasungsdauer. Die Steuerungseinheit steht dazu in, insbesondere bidirektionaler, Signalverbindung, insbesondere mit dem Heizelement, dem Gasmengenregulierer, dem Dampfgenerator, dem Dampfmengenregulierer und/oder einer Temperiereinheit.

Eine Temperiereinheit gemäß Anspruch 11 gewährleistet konstante Temperaturen in der Testkammer, insbesondere an einer Innenseite des Gehäuses der Testkammer. Die Temperiereinheit dient zum Temperieren einer Außenwand des Gehäuses. Die Temperiereinheit ist außerhalb und insbesondere unmittelbar an der Außenwand des Gehäuses angeordnet. Es wurde gefunden, dass dadurch Temperatureinflüsse auf die Testmenge innerhalb der Testkammer infolge von Wandeinflüssen reduziert und insbesondere vermieden werden können. Insbesondere weist die Testmenge in der Testkammer eine homogene Temperaturverteilung auf. Insbesondere kann mittels der Temperiereinheit das Risiko minimiert werden, dass ein Temperaturgefälle zu der Außenwand der Testkammer hin entsteht. Ein Temperaturunterschied zwischen der Testmenge und der Außenwand der Testkammer ist minimiert und beträgt höchstens 30 K, insbesondere höchstens 20 K, insbesondere höchstens 10 K und insbesondere höchstens 5 K.

Besonders vorteilhaft ist die Ausgestaltung der Temperiereinheit mit einem doppelwandigen Gehäuse in Form eines die Testkammer umgebenden Außenrohrs. Das Außenrohr kann mit einem erwärmten Fluid und insbesondere mit erwärmten Gas, insbesondere mit erwärmter Luft befüllt werden. Insbesondere ist an das doppelwandige Gehäuse die Druckgasleitung zum Zuführen von erwärmtem Druckgas angeschlossen. Bei dieser Ausgestaltung der Temperiereinheit kann insbesondere sichergestellt werden, dass das Außenrohr der Testkammer dieselbe Temperatur aufweist wie das in die Testkammer zugeführte Schüttgut. An das doppelwandige Gehäuse kann eine weitere Entlüftungsöffnung mit weiterer Entlüftungsleitung angeschlossen sein. Die weitere Entlüftungsöffnung mit der weiteren Entlüftungsleitung bilden eine Nebenentlüftung. Die weitere Entlüftungsleitung ist für den Transportzustand insbesondere demontierbar. Der über die Nebenentlüftung abgegebene Dampf ist insbesondere gesundheitlich unbedenklich. Über die Nebenentlüftung werden insbesondere heiße Luft und/oder reiner Wasserdampf abgeführt.

Anstelle der doppelwandigen Ausführung des Gehäuses kann die Temperiereinheit eine separate Heizung aufweisen, um die Außenwand des Gehäuses der Testkammer zu erwärmen. Diese separate Heizung, insbesondere in Form einer Heizdecke, kann unmittelbar an der Außenwand des Gehäuses der Testkammer angebracht sein.

Die separate Heizung, die insbesondere mit elektrischem Strom betrieben wird, kann insbesondere auch zum Vorwärmen der Testkammer genutzt werden, um insbesondere die Testergebnisse bei einer erstmaligen Durchführung der Entgasung und/oder Desodorierung, also bei einem sogenannten Ersttest, zu verbessern.

Eine Vorrichtung gemäß Anspruch 12 ermöglicht einen flexiblen und unkomplizierten Einsatz der Vorrichtung, insbesondere einen mobilen Einsatz an verschiedenen Orten. Die Vorrichtung ist mit der Abstellfläche unmittelbar auf einem Untergrund abstellbar. Dazu kann das Gestell nivellierbar ausgeführt sein, also insbesondere mindestens einen oder mehrere höhenverstellbar angeordnete Stützfüße aufweisen. Das Gestell ist insbesondere rahmenartig ausgeführt und umfasst insbesondere miteinander verbundene, insbesondere verschweißte Metallträger, insbesondere in Form von standardisierten Profilelementen. Das Gestell ist robust und kostengünstig ausgeführt. Insbesondere sind an dem Gestell alle wesentlichen Komponenten, insbesondere die Testkammer, befestigt. Vorteilhaft ist die lösbare Befestigung zu Reparatur- und Wartungszwecken. Vorteilhaft ist die Ausgestaltung des Gestells mit entsprechenden Aufnahmen für einen Gabelstapler und/oder Hubwagen. Der mobile Transport der Vorrichtung ist dadurch vereinfacht. Zusätzlich oder alternativ können seitlich und/oder an einer der Abstellfläche gegenüberliegenden Oberseite des Gestells Hebeösen befestigbar und/oder befestigt sein, um die Vorrichtung mittels eines Lastenkrans beispielsweise in einer Maschinenhalle vereinfacht zu transportieren. Denkbar ist auch, dass an der Abstellfläche mehrere Rollen angebracht sind, um die Vorrichtung mit dem Gestell unmittelbar auf dem Untergrund zu transportieren, wobei mindestens eine oder mehrere Rollen drehantreibbar ausgeführt sein können.

Eine Auswerteinheit gemäß Anspruch 13 ermöglicht die, insbesondere automatiserte, Verifizierung der an einer Testmenge angewendeten Betriebsparameter für das Entgasen und/oder Desodorieren. Die Auswerteeinheit dient zum Bestimmen von Materialeigenschaften und insbesondere der flüchtigen Bestandteile der Testmenge. Die Auswerteeinheit umfasst insbesondere einen Gaschromatographen, um den Anteil der flüchtigen Bestandteile in dem Schüttgut zu bestimmen.

Eine Verbindungsleitung gemäß Anspruch 14 vereinfacht die kombinierte Zugabe von erhitztem Druckgas mit Wasserdampf in die Testkammer. Eine entlang einer Verbindungsleitung angeordnete Regeleinheit dient zum Einstellen eines Verhältnisses von erwärmtem Druckgas, insbesondere erwärmter Druckluft, und zugeführter Feuchte. Mit der Regeleinheit kann insbesondere der Gasstrom, insbesondere der Luftstrom für das Entgasen in der Testkammer reguliert werden.

Ein Verfahren gemäß Anspruch 15 weist im Wesentlichen die Vorteile der Vorrichtung auf. Eine in die Testkammer eingefüllte Testmenge wird entgast und/oder desodoriert, indem der Testkammer ein temperiertes und/oder befeuchtetes Druckgas zugeführt wird. Nach einer veränderlich vorgebbaren Dauer wird, insbesondere wiederholt und insbesondere in regelmäßigen Zeitabständen, eine Probemenge über die Entnahmeöffnung aus der Testkammer entnommen. Für diese Probemenge werden dessen Materialeigenschaften, insbesondere die flüchtigen Bestandteile, bestimmt. Anhand von Veränderungen der Materialeigenschaften können die erforderlichen Betriebsparameter eingestellt und/oder ermittelt werden. Sofern die vorgegebenen Materialeigenschaften erreicht worden sind, können die mit der Vorrichtung durchgeführten Betriebsparameter auf die Compoundier- und/oder Recyclinganlage übertragen werden. Andernfalls wird eine neue Versuchsreihe gestartet mit veränderten Betriebsparametern. Das Verfahren wird insbesondere solange wiederholt, bis die entsprechenden Materialeigenschaften des Schüttguts erreicht worden sind.

Sowohl die in den Patentansprüchen angegebenen Merkmale als auch die in den nachfolgenden Ausführungsbeispielen der erfindungsgemäßen Vorrichtung angegebenen Merkmale sind jeweils für sich alleine oder in Kombination miteinander geeignet, den erfindungsgemäßen Gegenstand weiterzubilden. Die jeweiligen Merkmalskombinationen stellen hinsichtlich der Weiterbildungen des Erfindungsgegenstands keine Einschränkung dar, sondern weisen im Wesentlichen lediglich beispielhaften Charakter auf.

Weitere Merkmale, Vorteile und Einzelheiten der Erfindung ergeben sich aus der nachfolgenden Beschreibung von Ausführungsbeispielen anhand der Zeichnung. Es zeigen:

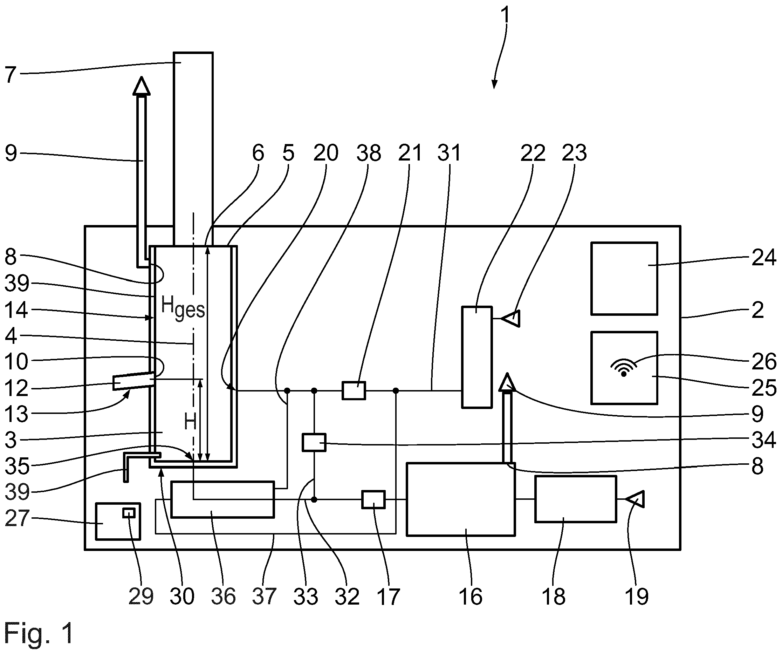

- Fig. 1: eine schematische Darstellung einer erfindungsgemäßen Vorrichtung.

Eine in Fig. 1 insgesamt mit 1 gekennzeichnete Vorrichtung umfasst ein Gestell 2, das in Fig. 1 rein schematisch dargestellt ist. Das Gestell 2 weist eine Abstellfläche auf, mit der das Gestell 2 und damit die Vorrichtung 1 insgesamt auf einem Untergrund zuverlässig und stabil anordenbar ist. Das Gestell 2 kann flächig, insbesondere plattenartig ausgeführt sein. Die Abstellfläche ist insbesondere viereckig, insbesondere rechteckförmig. Das Gestell 2 kann auch rahmenartig ausgeführt sein und mehrere miteinander verbundene, insbesondere verschweißte Längsträger aufweisen. Die Längsträger sind insbesondere als standardisierte Profilelemente, insbesondere als I- oder T-Träger aus Stahlmaterial hergestellt.

Das Gestell 2 kann an seiner Abstellfläche in an sich bekannter Weise höhenverstellbare Stützfüße und/oder Rollen aufweisen. Zusätzlich kann das Gestell 2 Gabelstapleraufnahmen und/oder Hebeösen aufweisen, um das Gestell 2 vereinfacht zu transportieren. Die Vorrichtung 1 eignet sich für den mobilen Einsatz.

An der Vorrichtung 1 ist eine Testkammer 3 lösbar befestigt. Die Testkammer 3 ist im Wesentlichen zylindrisch ausgeführt mit einer Längsachse 4, die insbesondere quer und insbesondere senkrecht zur Aufstellfläche des Gestells 2 orientiert ist. Insbesondere ist die Testkammer 3 mit der Längsachse 4 vertikal orientiert.

Die Testkammer 3 weist ein Gehäuse 30 auf, das doppelwandig ausgeführt ist.

In einem oberen Bereich, insbesondere an der oberen Stirnseite 5 der Testkammer 3 ist eine Entlüftungsöffnung 6 an der Testkammer 3 angeordnet. An die Entlüftungsöffnung 6 ist eine Entlüftungsleitung 7 angeschlossen. Mit der Entlüftungsleitung 7 ist die Testkammer 3 an eine zentrale Entlüftung anschließbar. Eine Einfüllöffnung zum Einfüllen der Kunststoffpartikel in die Testkammer 3 kann insbesondere durch die Entlüftungsöffnung 6 ausgebildet sein. Es ist beispielsweise möglich, die Entlüftungsleitung 7 von der Entlüftungsöffnung 6 zu entfernen, um Kunststoffgranulat durch die Entlüftungsöffnung 6 der Testkammer 3 zuzugeben.

Entsprechend ist eine Entleerung der Testkammer 3 über die obere Entlüftungsöffnung 6 möglich, beispielsweise mittels einer Absaugung. Alternativ kann eine separate Entleerungsöffnung an der Testkammer 3 vorgesehen sein, insbesondere im Bereich der unteren Stirnwand, also am Boden der Testkammer 3.

Zusätzlich ist eine weitere Entlüftungsöffnung 8 mit einer weiteren Entlüftungsleitung 9 an dem doppelwandigen Gehäuse 30 angeordnet. Die Entlüftungsöffnung 6 und die Entlüftungsleitung 7 haben einen ersten Leitungsquerschnitt, der größer ist als ein zweiter Leitungsquerschnitt der Entlüftungsöffnung 8 und der Entlüftungsleitung 9. Die Entlüftungsleitung 7 bildet eine Hauptentlüftung. Die Entlüftungsleitung 9 bildet eine Nebenentlüftung.

Die Testkammer 3 weist eine Entnahmeöffnung 10 auf, die insbesondere an der Zylindermantelwand der zylindrischen Testkammer 3 angeordnet ist. Bezogen auf eine der oberen Stirnseite 5 gegenüberliegend angeordneten unteren Stirnseite 11, die den Boden der Testkammer 3 bildet, ist die Entnahmeöffnung 10 in einer Höhenposition H angeordnet, die etwa der Hälfte der Gesamthöhe H_{ges} der Testkammer 3 entspricht. Insbesondere gilt: H ≤ 0,5 * H_{ges}, insbesondere H ≤ 0,4 * H_{ges} und insbesondere H ≤ 0,3 * H_{ges}. Durch die Höhenpositionierung der Entnahmeöffnung 10 an der Testkammer 3 ist gewährleistet, dass Schüttgut zuverlässig aus der Testkammer entnehmbar ist, insbesondere auch dann, wenn die Testkammer 3 nicht vollständig mit der Testmenge gefüllt ist.

An die Entnahmeöffnung 10 ist eine Entnahmeeinheit angeschlossen, die gemäß dem gezeigten Ausführungsbeispiel eine Entnahmeleitung 12 in Form eines, insbesondere zylindrischen, Fallrohrs umfasst. Die Entnahmeleitung 12 ist insbesondere gegenüber der vertikalen mit einem Neigungswinkel von weniger als 30 ° geneigt. Dadurch ist eine unkomplizierte, insbesondere schwerkraftbedingte Entnahme von Schüttgut aus der Testkammer 3 gewährleistet. Die Entnahmeeinheit weist ferner ein Absperrelement 13 auf, das insbesondere als Absperrschieber und/oder
- klappe, insbesondere in der Entnahmeleitung 12, ausgeführt sein kann. Mit dem Absperrelement 13 kann die Entnahmeleitung 12 unmittelbar abgesperrt und ein Schüttgutstrom hindurch verhindert werden.

Die Testkammer 3 weist eine Temperiereinheit 14 auf, die zum Temperieren einer Außenwand 39 der Testkammer 3 dient. Die Temperiereinheit 14 ist insbesondere unmittelbar an der Testkammer 3 angeordnet und durch ein doppelwandiges Gehäuse 30, also durch ein die Testkammer 3 umgebendes Außenrohr gebildet. Diesem die Testkammer 3 umgebenden Mantel kann ein Temperierfluid zugeführt werden, insbesondere erhitzte Luft. An das doppelwandige Gehäuse 30 ist an einem Druckgasanschluss 20 eine Druckgasleitung 31 angeschlossen, um insbesondere erwärmtes Druckgas, insbesondere erwärmte Druckluft, zuzuführen.

Die Testkammer 3 weist eine Zuführöffnung 35 auf, an die ein Dampfgenerator 16 mittels einer Dampfleitung 32 angeschlossen ist. Der an die Zuführöffnung 35 angeschlossene Abschnitt der Dampfleitung 32 wird auch als Begasungsleitung bezeichnet. Die Zuführöffnung 35 bildet einen Dampfanschluss an die Testkammer 3. Zusätzlich ist ein Dampfmengenregulierer 17 vorgesehen, der gemäß dem gezeigten Ausführungsbeispiel in dem Dampfgenerator 16 integriert angeordnet ist. Es ist alternativ möglich, dass der Dampfmengenregulierer 17 separat und insbesondere stromabwärts des Dampfgenerators 16 angeordnet ist. Mit dem Dampfmengenregulierer 17 wird die in die Testkammer 3 zugeführte Menge an Dampf reguliert. An dem Dampfgenerator 16 ist an einer Entlüftungsöffnung 8 eine Entlüftungsleitung 9 entsprechend der Nebenentlüftung 9 der Testkammer 3 angeschlossen.

Stromaufwärts des Dampfgenerators 16 ist eine optionale Wasserenthärtungseinheit 18 angeschlossen. Die Wasserenthärtungseinheit 18 ermöglicht die Nutzung von, insbesondere kalkhaltigem, Trinkwasser für die Dampferzeugung. An die Wasserenthärtungseinheit 18 ist stromaufwärts eine Wasserquelle 19 anschließbar. Die Wasserquelle 19 kann durch einen Wasseranschluss an ein Wasserleitungsnetz ausgeführt sein. Die Nutzung der Vorrichtung 1 ist dadurch vereinfacht. Insbesondere sind aufwendige Vorkehrungen für eine Wasserquelle entbehrlich.

Zusätzlich oder alternativ kann zu einem entsprechenden Wasserleitungsanschluss als Wasserquelle ein Wassertank vorgesehen sein. Mittels eines Wassertanks eignet sich die Vorrichtung für den autarken Einsatz. Insbesondere ist ein Wasserleitungsnetz für die Wasserzufuhr entbehrlich.

An die Druckgasleitung 31 ist ein Gasmengenregulierer 21, insbesondere ein Luftmengenregulierer angeschlossen. Stromaufwärts des Gasmengenregulierers 21 ist ein Heizelement 22, insbesondere ein Lufterhitzer angeordnet. An das Heizelement 22 ist eine Druckgasquelle 23 anschließbar. Die Druckgasquelle 23 kann ein Druckluftleitungsnetz sein, das typischerweise in einer Maschinenhalle ohnehin verfügbar ist. Zusätzlich oder alternativ kann die Druckgasquelle 23 als Kompressor ausgeführt sei. Mit dem Kompressor eignet sich die Vorrichtung für den mobilen, insbesondere autarken, Einsatz.

Die Druckgasleitung 31 ist mittels einer Verbindungsleitung 33 mit der Dampfleitung 32 verbunden. Entlang der Verbindungsleitung 33 ist eine Regeleinheit 34 angeordnet. Mittels der Verbindungsleitung 33 ist es möglich, insbesondere erhitztes, Druckgas von der Druckgasleitung 31 über die Verbindungsleitung 33 der Dampfleitung 32 zuzuführen und über die Begasungsleitung und die Zuführöffnung 35 der Testkammer 3 zuzuführen. In diesem Fall ist die Zuführöffnung 35 ein Begasungsanschluss.

Mit der Regeleinheit 34 kann der Anteil des erhitzten Druckgases, der der Dampfleitung 32 zugeführt wird, eingestellt werden. Mit den Regulierern 17, 21 und 34 ist es also möglich, entweder nur erhitztes Druckgas oder nur Wasserdampf oder beides in Kombination über die Zuführöffnung 35 in die Testkammer 3 zu zugegeben.

Die Vorrichtung 1 weist eine zentrale Spannungsversorgung 24 auf, die insbesondere fest mit der Vorrichtung 1 verbunden ist. Mittels der zentralen Spannungsversorgung 24 wird die für den Betrieb der Vorrichtung erforderliche Elektrizität bereitgestellt.

Die Vorrichtung 1 weist eine zentrale Steuerungseinheit 25 auf, die mittels einer in Fig. 1 rein symbolisch dargestellten Signalverbindung 26, insbesondere mit dem Dampfgenerator 16, dem Dampfmengenregulierer 17, dem Gasmengenregulierer 21, dem Gaserhitzer 22 und der Temperiereinheit 14 in, insbesondere bidirektionaler, Signalverbindung steht. Die Steuerungseinheit 25 ermöglicht die unkomplizierte und unmittelbare veränderliche Festlegung der Betriebsparameter für die Entgasung und Desodorierung der Testmenge in der Testkammer 3.

Die Vorrichtung 1 umfasst eine Auswerteinheit 27, die einen Gaschromatographen 29 umfasst. Die Auswerteeinheit 27 ist dazu geeignet, die Betriebsparameter für die Entgasung automatisiert zu bestimmen.

Die Auswerteeinheit 27 kann fest mit der Vorrichtung 1 verbunden sein. Die Auswerteinheit 27 kann alternativ mechanisch separiert von der Vorrichtung 1, insbesondere von dem Gestell 2 ausgeführt sein.

Entlang der Dampfleitung 32 ist stromabwärts des Dampfmengenregulierers 17 ein Isolierelement 36 angeordnet. Das Isolierelement 36 ist hohlförmig, insbesondere im Wesentlichen quaderförmig, ausgeführt. Das Isolierelement 36 ist kastenartig ausgeführt. Das Isolierelement 36 wird auch als Isolierkasten bezeichnet.

Die Begasungsleitung ist durch den Isolierkasten 36 durchgeführt, wobei ein Teilstrom über eine Abzweigleitung 37 im Kreuzstrom zu der Begasungsleitung durch den Isolierkasten 36 geführt wird. Die Abzweigleitung 37 ist eine Bypassleitung und dient zur Reduzierung von Wärmeverlusten des Gasstroms in die Testkammer 3. Das über die Abzweigleitung 37 in den Isolierkasten 36 zugeführte Gas wird über die Rückführleitung 38 aus dem Isolierkasten 36 abgeführt und der Druckgasleitung 31 zugeführt.

Eine weitere nicht dargestellte Bypassleitung, die im Vergleich zu der Bypassleitung 37 einen größeren Leitungsquerschnitt aufweist, kann dazu genutzt werden, die Probenkammer 3 mit erhitzter Luft und/oder Wasserdampf vorzuwärmen. Alternativ kann eine separate Heizung, insbesondere in Form einer Heizdecke, an der Außenwand 39 angebracht sein und zum Vorwärmen der Testkammer 3 genutzt werden.

An das Gehäuse 30 ist eine Drainageleitung 39 angeschlossen. Über die Drainageleitung 39 kann in dem doppelwandigen Gehäuse 30 gebildetes Kondensat gezielt abgeführt und insbesondere schwerkraftbedingt selbsttätig abgelassen werden. Die Drainageleitung 39 kann mit einem Sperrelement abschließbar ausgeführt sein.

Nachfolgend wird ein Verfahren zum Ermitteln der Betriebsparameter für das Entgasen und/oder Desodorieren einer Testmenge näher erläutert.

Bei der bereitgestellten Vorrichtung 1 wird in die Testkammer 3 eine Testmenge mit definierter Menge, beispielsweise 101, eingefüllt. Anschließend wird die Testmenge in der Testkammer mit definierten Betriebsparametern entgast und/oder desodoriert, also insbesondere mit einem ersten Druck, der von dem Gasmengenregulierer 21 bereitgestellt wird, einer ersten Temperatur, die von dem Heizelement 22 bereitgestellt wird, einer definierten Gasmenge, die mittels des Gasmengenregulierers 21 bereitgestellt wird, einer definierten Feuchte, die mittels des Dampfmengenregulierers 17 bereitgestellt wird und/oder über eine definierte Entgasungsdauer, die insbesondere auch über die zentrale Steuerungseinheit 25 vorgegeben wird, beaufschlagt. Insbesondere wird die Testmenge in der Testkammer 3 mit erhitztem Gas und/oder unter Dampfzugabe entgast und/oder desodoriert.

Nach Abschluss des Entgasungs- und/oder Desodorierungs-Zyklus wird Schüttgut, insbesondere eine Teilmenge der Testmenge, aus der Testkammer 3 mittels der Entnahmeleitung 12 entnommen und mittels der Auswerteeinheit 27 analysiert. Mit der Auswerteeinheit 27 werden vorgebbare Materialeigenschaften untersucht. Dazu weist die Auswerteinheit insbesondere einen Geruchssensor 28 und einen Gaschromatographen 29 auf.

In Abhängigkeit der ermittelten Materialeigenschaften kann ein neuer Entgasungs- und/oder Desodorierungs-Zyklus durchgeführt oder die zuvor angewendeten Betriebsparameter als geeignet qualifiziert werden.

Das Verfahren ist unkompliziert, unaufwendig und wenig kostenintensiv.

## Patentansprüche

1. Vorrichtung zum Ermitteln von Betriebsparametern für das Entgasen und/oder Desodorieren von Kunststoffpartikeln, wobei die Vorrichtung (1) mindestens eine Testkammer (3) zum Entgasen und/oder Desodorieren einer Testmenge von Kunststoffpartikeln umfasst, wobei jede Testkammer (3) aufweist
a. eine Einfüllöffnung zum Einfüllen der Testmenge,
b. eine Entnahmeöffnung (10) zum Entnehmen einer Probemenge der Kunststoffpartikel,
c. eine Entlüftungsöffnung (6) zum Entlüften der Testkammer (3), wobei an eine Zuführöffnung (35) der Testkammer (3) angeschlossen sind,
d. eine Druckgasleitung (31) zum Zuführen von Druckgas und ein Heizelement (22) und/oder,
e. eine Dampfleitung (32) zum Zuführen von Dampf und ein Dampfgenerator (16).

2. Vorrichtung gemäß Anspruch 1, **gekennzeichnet durch** einen an die Druckgasleitung (31) angeschlossenen Gasmengenregulierer (21).

3. Vorrichtung gemäß einem der vorstehenden Ansprüche, **gekennzeichnet durch** eine mit der Druckgasleitung (31) verbindbare Druckgasquelle (23).

4. Vorrichtung gemäß einem der vorstehenden Ansprüche, **dadurch gekennzeichnet, dass** der Dampfgenerator (16) eine Entlüftungsöffnung (8) aufweist.

5. Vorrichtung gemäß einem der vorstehenden Ansprüche, **gekennzeichnet durch** eine an den Dampfgenerator (16) angeschlossene Wasserenthärtungseinheit (18).

6. Vorrichtung gemäß einem der vorstehenden Ansprüche, **gekennzeichnet durch** einen an die Dampfleitung (32) angeschlossenen Dampfmengenregulierer (17).

7. Vorrichtung gemäß einem der vorstehenden Ansprüche, **gekennzeichnet durch** eine an den Dampfgenerator (16) anschließbare Wasserquelle (19).

8. Vorrichtung gemäß einem der vorstehenden Ansprüche, **gekennzeichnet durch** eine an die Entlüftungsöffnung (6) anschließbare Entlüftungsleitung (7) zum Verbinden der Testkammer (3) mit einem zentralen Entlüftungssystem.

9. Vorrichtung gemäß einem der vorstehenden Ansprüche, **gekennzeichnet durch** eine an die eine Entnahmeöffnung (10) angeschlossene Entnahmeeinheit, die insbesondere ein mittels eines Absperrelements (13) absperrbare Entnahmeleitung (12), insbesondere ein Fallrohr, umfasst.

10. Vorrichtung gemäß einem der vorstehenden Ansprüche, **gekennzeichnet durch** eine Steuerungseinheit (25), die dazu ausgebildet ist, Betriebsparameter, insbesondere Temperatur, Druck und/oder Menge des zugeführten Druckgases, Wassergehalt und/oder Entgasungsdauer für das Entgasen und/oder Desodorieren der Testmenge in der Testkammer (3) zu steuern.

11. Vorrichtung gemäß einem der vorstehenden Ansprüche, **gekennzeichnet durch** eine Temperiereinheit (14) zum Temperieren einer Außenwand (39) der Testkammer (3), wobei die Temperiereinheit (14) ein die Testkammer (3) umgebendes doppelwandiges Gehäuse (30) oder eine separate Heizung aufweist.

12. Vorrichtung gemäß einem der vorstehenden Ansprüche, **gekennzeichnet durch** ein die Vorrichtung (1) tragendes Gestell (2), an dem insbesondere die Testkammer (3) befestigt ist.

13. Vorrichtung gemäß einem der vorstehenden Ansprüche, **gekennzeichnet durch** eine Auswerteeinheit (27) zum, insbesondere automatisierten, Bestimmen von Betriebsparametern, wobei die Auswerteeinheit (27) insbesondere einen Gaschromatographen (29) umfasst.

14. Vorrichtung gemäß einem der vorstehenden Ansprüche, **dadurch gekennzeichnet, dass** die Druckgasleitung (31) und die Dampfleitung (32) mittels einer Verbindungsleitung (33) unmittelbar verbunden sind, wobei insbesondere entlang der Verbindungsleitung (33) eine Regeleinheit (34) angeordnet ist.

15. Verfahren zum Ermitteln von Betriebsparametern für das Entgasen und/oder Desodorieren von Kunststoffpartikeln umfassend die Verfahrensschritte
- Bereitstellen einer Vorrichtung (1) gemäß einem der vorstehenden Ansprüche,
- Einfüllen einer Testmenge von Kunststoffpartikeln in die Testkammer (3),
- Entgasen und/oder Desodorieren der Testmenge in der Testkammer (3) durch Zuführen von temperiertem Druckgas und/oder von Dampf über eine Zuführöffnung (35) der Testkammer (3),
- Entnehmen einer Probenmenge der entgasten und/oder desodorierten Kunststoffpartikel aus der Testkammer (3),
- Bestimmen von Materialeigenschaften der Probenmenge,
- Ermitteln von Betriebsparametern für das Entgasen und/oder Desodorieren auf Basis der bestimmten Materialeigenschaften.
